Europäisches Patentamt

European Patent Office

Office européen des brevets

(19)

(11) Veröffentlichungsnummer: **0 347 640**

**A1**

(12) # EUROPÄISCHE PATENTANMELDUNG

(21) Anmeldenummer: **89110180.0**

(22) Anmeldetag: **06.06.89**

(51) Int. Cl.⁴: **A61B 5/00 , A61B 5/10**

(30) Priorität: **23.06.88 DE 3821219**

(43) Veröffentlichungstag der Anmeldung:
**27.12.89 Patentblatt 89/52**

(84) Benannte Vertragsstaaten:
**CH GB IT LI SE**

(71) Anmelder: **PHYWE SYSTEME GMBH**
**Robert-Bosch-Breite 10**
**D-3400 Göttingen(DE)**

(72) Erfinder: **Hermsdörffer, Joachim, Dipl.-Ing.**
**Herzog-Heinrich-Strasse 30**
**D-8000 München 2(DE)**
Erfinder: **Galfe, Günther, Dipl.-Ing.**
**Fraunhoferstrasse 14**
**D-8000 München 5(DE)**
Erfinder: **Lautenbacher, Stefan, Dipl.-Psych.**
**Spixstrasse 4**
**D-8000 München 90(DE)**
Erfinder: **Korte, Reinhard, Dipl.-Ing.**

**Bebelstrasse 42**
**D-3400 Göttingen(DE)**
Erfinder: **Krause, Hans-Joachim, Ing. (grad.)**
**Am Burggraben 25**
**D-3400 Göttingen/Elliehausen(DE)**
Erfinder: **Krüll, Frans, Dr. Dipl.-Biol.**
**Südring 85**
**D-3400 Göttingen(DE)**
Erfinder: **May, Arno, Dr. Dipl.-Phys.**
**Ludwig-Prandtl-Strasse 41**
**D-3400 Göttingen(DE)**
Erfinder: **Oberdorfer, Dietmar, Konstr.**
**Brüsselstrasse 13 D**
**D-3400 Göttingen(DE)**
Erfinder: **Plüquett, Ulrich, Phys.**
**Kalklage 1e**
**D-3400 Göttingen(DE)**

(74) Vertreter: **Rehberg, Elmar, Dipl.-Ing.**
**Postfach 3162 Am Kirschberge 22**
**D-3400 Göttingen(DE)**

(54) **Stimulationsvorrichtung.**

(57) Stimulationsvorrichtung zur Bestimmung des Temperaturempfindens der Haut eines Patienten, mit einem in einem Gehäuse (4) angeordneten Reizgeber (1), der über ein Peltier-Element (2) aufheizbar bzw. abkühlbar ist. Der Reizgeber (1) ist in dem Gehäuse (4) entlang einer Achse (5) beweglich angeordnet und weist in Ruhestellung gegenüber dem Gehäuse (4) einen Überstand (14) auf. Es ist eine erste Andruckeinrichtung (6) vorgesehen, die mit dem Reizgeber (1) derart zusammenwirkt, daß der Reizgeber (1) unter Aufbringung einer Kraft in Richtung der Achse (5) solange verschiebbar ist, bis der Überstand (14) zu Null wird. Die Kraft ist durch die Andruckeinrichtung (6) fest vorgegeben oder einstellbar.

Fig. 1

## Stimulationsvorrichtung

Die Erfindung geht aus von einer Stimulationsvorrichtung zur Bestimmung des Temperatur- und Schmerzempfindens eines Patienten mittels kutaner Kontaktstimulation, mit einem in einem Gehäuse angeordneten Reizgeber, der über ein Peltier-Element aufheizbar bzw. abkühlbar ist.

Die Untersuchung des Temperatur- und Schmerzempfindens dient bei zahlreichen Erkrankungen, wie beispielsweise der diabetischen Neuropathie, der Frühdiagnostik sowie der Überprüfung der Krankheits- und Therapieverläufe. Untersucht werden das Warm-Kalt-Empfinden sowie die Wahrnehmungsfähigkeit für Hitzeschmerz, die beide den Funktionsstatus kleiner Nervenfasern wiederspiegeln. Der Reizgeber wird mit einer geeigneten Körperstelle des Patienten in Kontakt gebracht. Die Temperaturen des Reizgebers werden, jeweils von einer mittleren Temperatur ausgehend, erhöht oder gesenkt. Der Patient meldet, wenn er eine Temperaturänderung bemerkt (Warm-Kalt-Empfindung) oder wenn er die erste Schmerzempfindung wahrnimmt (Schmerzempfindung). Bei einem Patienten mit beispielsweise diabetischer Neuropathie ist sowohl die Warm-Kalt-Empfindung als auch die Scherzempfindung zu höheren Werten verlagert. Dies trifft auch bei anderen Erkrankungen mit Schädigung der dünnen Nervenfasern zu, so daß in diesen Fällen mit der genannten Methode Diagnose und Therapievalution möglich ist.

Eine Stimulationsvorrichtung der eingangs beschriebenen Art ist aus einer Werbeschrift der Firma Medelec Ltd., betitelt "TTT" bekannt. Der Reizgeber ist in dem Gehäuse angeordnet und fest mit dem Gehäuse verbunden. Das Peltier-Element wird, entsprechend der Richtung des dem Peltier-Element zugeführten Stroms, aufgeheizt bzw. abgekühlt. Der Reizgeber steht mit dem Peltier-Element derart in Verbindung, daß die Wärme auf den Reizgeber entsprechend übertragen wird. Die Stimulationsvorrichtung wird mit einer geeigneten Körperstelle des Patienten, beispielsweise der Hand oder dem Fuß, in Kontakt gebracht. Bei den sich daran anschließenden Untersuchungen hat es als nachteilig herausgestellt, daß die Untersuchungsergebnisse vom Auflagedruck des Stimulators abhängig sind. Der Auflagedruck ist bei den bisher bekannten Vorrichtungen nicht exakt einstellbar. Dies erhöht den Meßfehler und verringert daher die Meßzuverlässigkeit. Die DE-OS 33 09 093 zeigt ein Gerät zur Messung der Durchblutungsfunktion der Haut, wobei eine gewisse Hautpartie aufgeheizt wird und der Temperaturverlauf an einer nicht aufgeheizten Stelle inmitten des beheizten Gebiets gemessen wird. Die Vorrichtung weist ein Gehäuse auf, in welches ein fluidbetätigter Kolben, abgestützt auf einer Rückholfeder, beweglich angeordnet ist, der eine Baugruppe aus dem Peltier-Element trägt, so daß auf diese Art und Weise die Anlage des Peltier-Elements an der Haut des Patienten möglich ist. Die auf das Gehäuse einwirkende Reaktionskraft des pneumatischen Drucks setzt voraus, daß das Gewicht des Gehäuses entsprechend groß ist, damit sich diese Reaktionskraft ausbilden kann und nicht zu einem Abheben des Gehäuses von der Haut des Patienten führt. Diese Konstruktion setzt weiterhin voraus, daß das Gehäuse nur von oben senkrecht nach unten weisend auf die Haut des Patienten aufgesetzt werden kann. Eine umgekehrte Gebrauchslage oder gar eine Gebrauchslage an einer senkrechten Stelle des Patienten, beispielsweise an der Wange des Patienten oder im Fußbereich, ist nicht möglich. Auf jeden Fall aber wird mit dem nicht unbeträchtlichen Gewicht des Gehäuses der Patient belastet, so daß die Gefahr besteht, daß der zu untersuchende Bereich durch diese Gewichtskraft abgeschnürt wird, so daß aus diesem Grund bereits eine geringere Durchblutung des betr. Bereichs stattfindet, der das Meßergebnis natürlich verfälscht.

Die DE-OS 29 12 349 zeigt eine Vorrichtung zur Bestimmung des Feuchtigkeitszustands der menschlichen Haut, wobei dort ein Elektrodenhalter gegenüber einem Gehäuse mit Überstand auf einer Feder gelagert angeordnet ist und entgegen der Kraft der Feder auf die Haut des Patienten aufsetzbar und andrückbar ist, bis der Überstand verschwunden ist. Das Andrücken an der menschlichen Haut geschieht offensichtlich per Hand und ist damit bezüglich der Anpreßkraft variabel und nicht reproduzierbar.

Der Erfindung liegt daher die Aufgabe zugrunde, eine Stimulationsvorrichtung der eingangs beschriebenen Art so weiterzubilden, daß weitgehend reproduzierbare Untersuchungsergebnisse in allen Gebrauchslagen erzielbar sind.

Erfindungsgemäß wird dies bei der Stimulationsvorrichtung der eingangs beschriebenen Art dadurch erreicht, daß der Reizgeber in dem Gehäuse entlang einer Achse beweglich angeordnet ist und in Ruhestellung gegenüber einer Auflagefläche des Gehäuses einen Überstand aufweist, daß eine erste Andruckeinrichtung für den Reizgeber und eine zweite Andruckeinrichtung für die Auflagefläche des Gehäuses vorgesehen ist, daß die erste Andruckeinrichtung mit dem Reizgeber derart zusammenwirkt, daß der Reizgeber unter Aufbringung einer Kraft in Richtung der Achse solange verschiebbar ist, bis der Überstand zu Null wird, wobei die Kraft über die erste Andruckeinrichtung einstellbar ist, und daß die zweite Andruckeinrichtung mit dem Gehäuse derart zusammenwirkt, daß die Auflagefläche des Gehäuses unter Aufbringung einer zweiten einstellbaren Kraft in Richtung der Achse feststellbar ist. Der Reizgeber ist also hier nicht mehr fest mit dem Gehäuse verbunden, sondern entlang einer Achse beweglich angeordnet. In der Ruhestellung, d h. wenn der Reizgeber nicht in Kontakt mit einer Körperstelle des Patienten gebracht ist, weist der Reizgeber einen Überstand gegenüber dem Gehäuse auf, ragt also um einen bestimmten Betrag aus dem Gehäuse heraus. Die Andruckeinrichtung ist mit dem Reizgeber derart verbunden, daß beim Verschieben des Reizgebers in Richtung der Achse eine bestimmte Kraft aufgewendet werden muß. Dabei ist die aufzuwendende Kraft durch die erste Andruckeinrichtung einstellbar. Bei Verwendung der Stimulationsvorrichtung wird das Gehäuse mit der ausgewählten Körperstelle des Patienten in der jeweiligen Gebrauchslage, also in waagerechter oder senkrechter Anordnung oder in Schräglage, über die zweite Andruckeinrichtung mit der Haut des Patienten in Kontakt gebracht. Dabei ist wichtig, daß das Gehäuse der Stimulationsvorrichtung auf der Haut des Patienten gerade anliegt bzw. daß von dem Gehäuse nur ein relativ geringer Druck auf die Haut aufgebracht wird. Der Reizgeber wird dann von der Andruckeinrichtung mit der vorgegebenen Kraft an die Haut des Patienten gedrückt. Damit wird sichergestellt, daß der Reizgeber bei allen Untersuchungen, unabhängig von der Körperstelle des Patienten, mit dem vorgegebenen Druck auf der Haut des Patienten aufliegt. Dieser reproduzierbare Auflagedruck hat sich als eine wichtige Voraussetzung zum Erhalten reproduzierbarer Untersuchungsergebnisse herausgestellt. In Abhängigkeit von dem Auflagedruck werden neben den thermosensiblen Nervenfasern mechanosensible durch die Druckkräfte mehr oder weniger stark aktiviert. Diese Kostimulation kann Einfluß auf das Temperatur- und Schmerzempfinden ausüben. Dadurch bedingt ändert sich auch das Temperaturempfinden, welches, wie oben beschrieben, als Maß für diagnostische Zwecke herangezogen wird. Ebenfalls ist der Wärmeübergang von dem Reizgeber auf die Haut abhängig von dem Auflagedruck. Bei der im Stand der Technik bekannten Stimulationsvorrichtung ist der Auflagedruck abhängig von einer Mehrzahl von Parametern, so daß letztendlich die Untersuchungsergebnisse aufgrund der Abhängigkeit des Auflagedrucks erheblich schwanken. Im Gegensatz wird bei der vorliegenden Erfindung der Auflagedruck stets konstantgehalten. Auch bei der Auswahl unterschiedlicher Körperstellen, also beispielsweise bei Messung des Temperaturempfindens einmal an der Hand und ein anderes Mal an dem Fuß des Patienten bzw. bei verschiedenen Patienten, bei denen die Hände und Füße auch unterschiedliche Gestalt aufweisen, ist der konstante Auflagedruck stets gewährleistet.

Die erste Andruckeinrichtung kann eine Feder aufweisen, die sich einerseits an dem Reizgeber und andererseits an einem in dem Gehäuse angeordneten Anschlag abstützt. Die Verwendung der Feder, vorzugsweise als Druckfeder, hat den Vorteil, daß sie relativ einfach herstellbar ist bzw. daß sie käuflich zu einem günstigen Preis erworben werden kann. Dabei erfüllt sie die ihr zugeordnete Aufgabe, nämlich auf den Reizgeber eine bestimmte Kraft auszuüben, zuverlässig. Die Feder stützt sich einerseits an dem Reizgeber selber ab und andererseits an dem im Gehäuse angeordneten Anschlag. Dabei kann der sich im Gehäuse angeordnete Anschlag zur Verstellung der Größe der auf den Reizgeber wirkenden Kraft, vorzugsweise koaxial zur Bewegungsrichtung des Reizgebers, verstellbar sein. Diese Verstellung kann durch eine einfache Rändelschraube erfolgen, deren Kopf aus dem Gehäuse geführt ist. Die Rändelschraube wirkt dann mit dem Anschlag derart zusammen, daß durch das Drehen der Rändelschraube der Anschlag koaxial zur Bewegungsrichtung des Reizgebers verschoben wird. Damit kann dann die Federspannung und somit letztendlich auch die Größe der auf den Reizgeber wirkenden Kraft den jeweiligen Verhältnissen angepaßt werden. Um die einmal eingestellte Kraft auch später wieder reproduzieren zu können, kann eine die auf den Reizgeber wirkende Kraft bzw. die Stellung des Anschlags wiedergebende Anzeigeeinrichtung vorgesehen sein. Die Anzeigeeinrichtung kann dann beispielsweise ein in dem Gehäuse angeordnetes Fenster aufweisen, durch die dann eine entsprechende Markierung, an der ein an dem Anschlag angeordneter Zeiger vorbeiläuft, abgelesen werden kann.

Die zweite Andruckeinrichtung kann einen in allen Richtungen schwenk- und verstellbaren Haltearm aufweisen, wobei das Gehäuse an der zweiten Andruckeinrichtung gelagert ist. Das eine Ende des Haltearms ist an einem feststehenden Gegenstand, beispielweise einem Tischbein o. dgl., befestigt. An dem anderen Ende des Haltearms ist das Gehäuse vorgesehen, wobei zwischen dem Haltearm und dem Gehäuse die zweite Andruckeinrichtung angeordnet ist. Die zweite Andruckeinrichtung sorgt dafür, daß das Gehäuse stets mit nahezu gleichbleibendem Druck auf der jeweiligen

Körperstelle des Patienten anliegt. Dies ist insoweit wichtig, da somit sichergestellt ist, daß das Gehäuse tatsächlich auf der Haut des Patienten anliegt und somit die stets gleichbleibende Druckbelastung des Reizgebers auf der Haut des Patienten vorliegt. Weiterhin kann auch durch den auf die Haut ausgeübten Gehäusedruck eine Beeinflussung des Untersuchungsergebnisses auftreten. Auch der Gehäusedruck trägt zur mechanischen Kostimulation bei und kann so zu Veränderungen des Temperatur- und Schmerzempfindens führen. Außerdem kann bei längeren Untersuchungen, insbesondere bei Patienten mit Durchblutungsstörungen, ein zu hoher Gehäusedruck die Blutzirkulation unter und neben dem Stimulator beeinträchtigen. Dies führt über Veränderungen der Temperatur des kutanen und subkutanen Gewebes zu Variationen in der Empfindungsstärke. Insoweit trägt die zweite Andruckeinrichtung in Verbindung mit dem Haltearm zu der Reproduzierbarkeit der Untersuchungsergebnisse bei.

Die zweite Andruckeinrichtung kann eine mit dem Gehäuse starr verbundene Welle aufweisen, wobei die Welle in einer an dem Haltearm befestigbaren Hülse beweglich geführt ist und wobei eine die Welle in einer vorzugsweise mittleren Position fixierenden Haltevorrichtung angeordnet sein kann. Die Haltevorrichtung kann derart ausgebildet und angeordnet sein, daß die Welle aus der fixierten Position unter Aufbringung einer Kraft verschiebbar ist. Die Haltevorrichtung kann vorzugsweise zwei Druckfedern aufweisen, wobei jeder Druckfeder eine Auflage an der Hülse und eine zweite Auflage an der Welle zugeordnet sind, und daß bei Belastung der einen Druckfeder die andere entlastet wird und umgekehrt. Die Haltevorrichtung kann eine Anzeige aufweisen, von der die Größe und die Richtung der auf den Druckfedern wirkenden Belastung ablesbar ist. Durch die Anordnung zweier Druckfedern ist gewährleistet, daß jeweils eine der beiden Druckfedern in Abhängigkeit davon, ob der Reizgeber über Kopf steht oder nicht, wirkt. Bevor das Gehäuse mit der Haut des Patienten in Kontakt gebracht wird, kann in der jeweiligen Position, also über Kopf oder nicht, schräg oder gerade, von der Anzeige die durch die Gewichtskraft hervorgerufene Kraft abgelesen werden. Anschließend wird das Gehäuse mit dem Reizgeber durch entsprechende Verstellung des Haltearms mit der Haut des Patienten in Kontakt gebracht und beispielsweise die eine Druckfeder durch Verschiebung der Hülse in Richtung des Gehäuses derart belastet, daß das Gehäuse mit einem bestimmten Auflagedruck auf der Haut des Patienten aufliegt. Der Auflagedruck ergibt sich aus dem angezeigten Wert vor der Auflage des Gehäuses auf die Haut in Verbindung mit dem nach der Auflage angezeigten Wert. Diese Anordnung hat auch den weiteren Vorteil, daß der Patient bei Überhitzung des Reizgebers durch beispielsweise einen Defekt nicht fest mit dem Reizgeber bzw. dem Gehäuse verbunden ist, sondern durch eine entsprechende Bewegung von diesem freikommen kann.

Als ein weiterer, wenn auch nicht derart ausschlaggebender Einflußfaktor auf die Untersuchungsergebnisse hat sich herausgestellt, daß das Temperaturempfinden abhängig von den für den Aufheizvorgang bzw. für die Kühlung benötigten Kühlraten ist. Aussagekräftige Ergebnisse erhält man bei möglichst stabilen Heiz- bzw. Kühlraten. Dabei ist insbesondere der Kühlvorgang technisch schwieriger zu realisieren. Um eine möglichst stabile Kühlrate auch im unteren Temperaturbereich in der Reizphase zu erhalten sowie eine Rücksetzung zur "Baseline" zur Wiederherstellung der Prästimulationsbedingungen zu ermöglichen, kann dem Peltier-Element ein flüssigkeitsgekühlter Wärmetauscher zugeordnet sein und ein zweites, die Flüssigkeit des Wärmetauschers kühlendes Peltier-Element vorgesehen sein. Durch die Anordnung eines Wärmetauschers, der flüssigkeitsgekühlt ist, wird die Kühlleistung bereits in erheblichem Maße gesteigert. Das zweite Peltier-Element ist derart angeordnet bzw. wird derart angesteuert, daß die kalte Seite des Peltier-Elements mit der warmen Seite des Wärmetauschers in Verbindung steht und somit die Kühlflüssigkeit abkühlt. Damit kann die für die Kühlung benötigte Zeit in etwa halbiert werden bzw. kann die Kühlleistung in Grad/sec. in etwa verdoppelt werden.

Die Erfindung wird anhand von bevorzugten Ausführungsbeispielen weiter beschrieben. Es zeigen:

Figur 1 ein Ausführungsbeispiel einer Stimulationsvorrichtung,

Figur 2 die Stimulationsvorrichtung gemäß Figur 1 nach Auflage auf die Haut eines Patienten,

Figur 3 einen Haltearm mit einer zweiten Andruckeinrichtung für die Stimulationsvorrichtung,

Figur 4 eine schematische Darstellung eines Kühlkreislaufs und

Figur 5 eine Leitungsführung des Kühlkreislaufs.

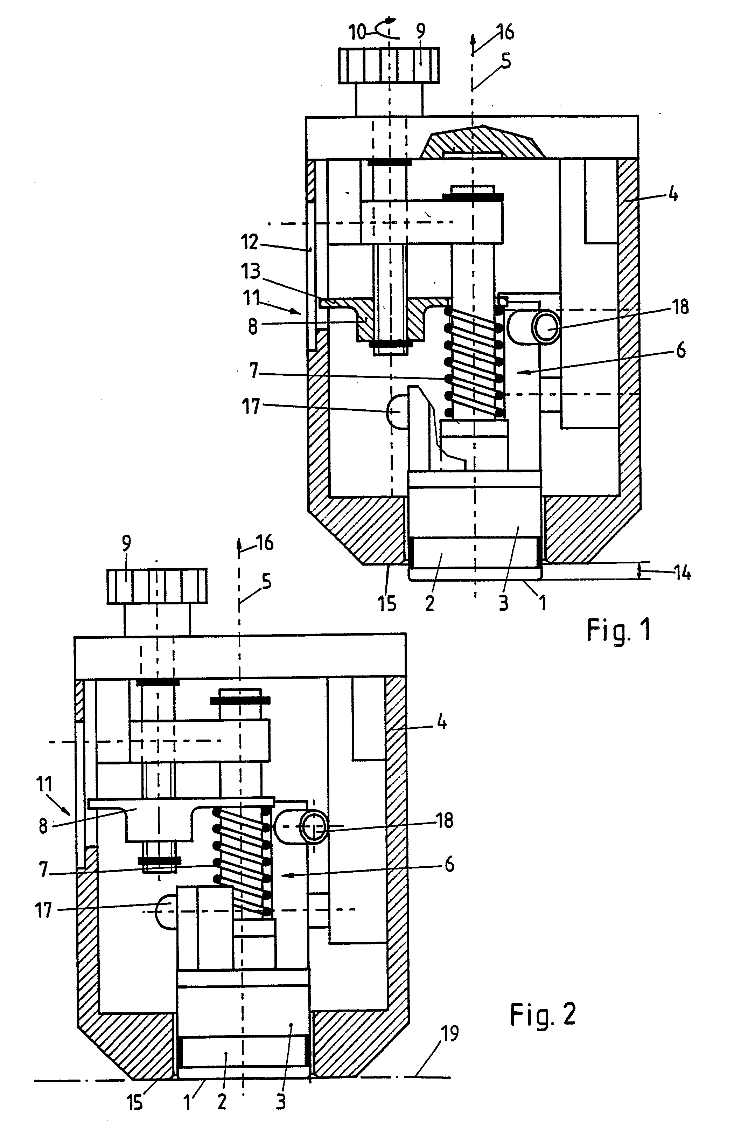

In Figur 1 ist eine erste Ausführungsform der erfindungsgemäßen Stimulationsvorrichtung dargestellt. Ein Reizgeber 1 ist mit einem Peltier-Element 2 leitend verbunden. Das Peltier-Element 2 wird von einem Wärmetauscher 3, der ebenfalls mit dem Peltier-Element in Verbindung steht, gekühlt. Der Reizgeber 1 und somit das Peltier-Element 2 und der Wärmetauscher 3 sind in einem Gehäuse 4 derart gelagert, daß der Reizgeber 1, das Peltier-Element 2 und der Wärmetauscher 3 entlang einer Achse 5 verschiebbar sind. Die Verschiebung erfolgt entgegen der Kraft einer Andruckeinrichtung 6, die eine Feder 7 und einen Anschlag 8 aufweist. Der Anschlag 8 ist mit Hilfe einer Rändelschraube 9 koaxial zu der Achse 5 verstellbar, so daß die Feder 7 einstellbar ist. Der Kopf der Rändelschraube 9 liegt außerhalb des Gehäuses 4, währenddessen das Gewindeteil der Rändelschraube 9 mit einem entsprechenden Gewinde mit dem Anschlag 8 zusammenarbeitet. Zum Verstellen des Anschlags 8 wird die Rändelschraube 9 entsprechend einem Pfeil 10 gedreht, wodurch der Anschlag 8 die Feder 7 entlastet. Bei Drehung der Rändelschraube 9 in entgegengesetzter Richtung des Pfeils 10 findet eine Belastung der Feder 7 statt. Eine Anzeigeeinrichtung 11, die im wesentlichen ein Fenster 12 und einen Zeiger 13 sowie eine hier nicht weiter dargestellte Markierung aufweist, dient zum Ablesen der jeweiligen Stellung des Anschlags 8 bzw. der eingestellten Spannung der Feder 7. Im unbelasteten Zustand, also wenn die Stimulationsvorrichtung noch nicht mit der Haut eines Patienten in Kontakt gebracht ist, weist der Reizgeber 1 einen Überstand 14 gegenüber dem Gehäuse 4 auf.

Im Folgenden wird nun die Funktionsweise der Stimulationsvorrichtung beschrieben. Zur Untersuchung des Temperaturempfindens wird die Stimulationsvorrichtung auf die Haut des hier nicht weiter dargestellten Patienten derart angedrückt, daß eine im wesentlichen ebene Anlagefläche 15 des Gehäuses 4 auf der Haut des Patienten aufliegt. Der Reizgeber 1 wird dabei in Richtung eines Pfeils 16 entgegen der Kraft der Feder 7 auf der Achse 5 verschoben. Die Feder 7 stützt sich einerseits an dem Anschlag 8 und andererseits an dem Reizgeber 1 bzw. dem Wärmetauscher 3 ab. Die Spannung der Feder 7 wird durch Drehen der Rändelschraube 9 verstellt, wobei die jeweilige Stellung bzw. die jeweilige Spannung der Feder 7 an der Anzeigeeinrichtung 11 ablesbar ist. Es ist somit sichergestellt, daß der Reizgeber 1 stets mit dem gleichen reproduzierbaren Druck auf der Haut des Patienten anliegt. Das nun erfolgende Aufheizen bzw. Abkühlen des Reizgebers 1 erfolgt durch entsprechende, hier nicht weiter dargestellte Stromzuführungen des Peltier-Elements 2. Um eine möglichst kurze Abkühlzeit bzw. eine möglichst große Abkühlrate zu erreichen, ist der Wärmetauscher 3 mit der warmen Seite des Peltier-Elements 2 wärmeleitend verbunden. Der Wärmetauscher 3 weist dabei in seinem Innern eine Vielzahl von Rippen auf, um einen möglichst guten Wärmeübergang zu erreichen. Dem Wärmetauscher 3 wird die Kühlflüssigkeit über eine Zufuhrleitung 17 zugeführt und von einer Abfuhrleitung 18 wieder abgeführt.

Die Stimulationsvorrichtung entsprechend der Figur 2 unterscheidet sich im wesentlichen von derjenigen der Figur 1 dadurch, daß mit Hilfe der Rändelschraube 9 eine andere Stellung des Anschlags 8 eingestellt ist, daß also die Spannung der Feder 7 verändert ist. Die Feder 7 ist hierbei in zusammengedrücktem Zustand gezeigt, also in demjenigen Zustand, bei dem die Anlagefläche 15 auf der Haut des Patienten anliegt und somit der Reizgeber 1 bzw. dessen äußere Oberfläche mit der Anlagefläche 15 in einer Ebene 19 zu liegen kommt. In dem hier dargestellten Zustand wird der Reizgeber 1 entgegen der Richtung des Pfeils 16 durch die Feder 7 mit einer an der Andruckeinrichtung 6 eingestellten Kraft beaufschlagt. Die Größe der Kraft ist durch Verstellen des Anschlags 8 mit Hilfe der Rändelschraube 9 variierbar, so daß je nach Betriebslage, also waagerechter, senkrechter Anordnung oder Schräglage, sowohl eine Kompensation des Eigengewichts der mit dem Reizgeber 1 verbundenen Teile erfolgt als auch eine Veränderung des Auflagedrucks des Reizgebers 1 in einem bestimmten Bereich möglich wird. Damit ist es möglich, einen stets reproduzierbaren Auflagedruck des Reizgebers 1 zu erreichen und den Auflagedruck auch den jeweiligen Verhältnissen anzupassen.

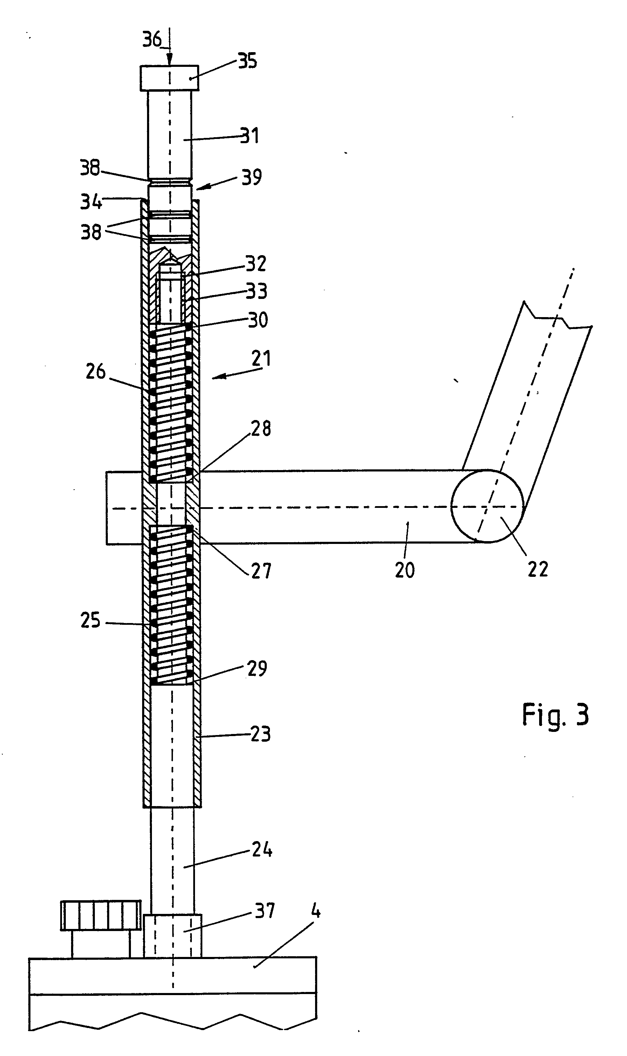

In Figur 3 ist der eine Teil eines Haltearms 20 gezeigt, an dem eine zweite Andruckeinrichtung 21 befestigt ist. Der Haltearm 20 weist eine Vielzahl von Gelenken 22 auf, so daß er insgesamt in allen Richtungen schwenk- und verstellbar ist. Die zweite Andruckeinrichtung 21 ist mit dem einen Ende des Haltearms 20 fest verbunden. Die zweite Andruckeinrichtung 21 weist eine Hülse 23 auf, in der eine Welle 24, die mit dem Gehäuse 4 fest verbunden ist, geführt wird. Es sind eine erste Druckfeder 25 und eine zweite Druckfeder 26 vorgesehen, die die Welle 24 in einer mittleren Position halten. Die Druckfedern 25 und 26 stützen sich dabei jeweils an einer Auflage 27 bzw. 28 der Hülse 23 und an Auflagen 29 bzw. 30 der Welle 24 ab. Zur Bildung der Auflage 29 ist der Durchmesser der Welle 24 reduziert, die Auflage 30 wird durch ein auf die Welle 24 aufschraubbares Verlängerungsteil 31 gebildet. Das Verlängerungsteil 31 weist dafür ein Gewinde 32 auf, welches mit einem entsprechenden Gegengewinde 33 der Welle 24 zusammenarbeitet. Die axiale Erstreckung des Verlängerungsteils 31 ist derart gewählt, daß das Verlängerungsteil 31 über einem Ende 34 der Hülse 23 hinausragt. An dem dem Gewinde 32 gegenüberliegenden Ende des Verlängerungsteils 31 ist ein Anschlag 35 vorgesehen, der die axiale Beweglichkeit der Welle 24 in der Hülse 23 in Richtung eines Pfeils 36 begrenzt. Die Begrenzung in Richtung entgegen des Pfeils 36 erfolgt durch einen zweiten Anschlag 37, der an dem Gehäuse 4 befestigt ist. Das Verlängerungsteil 31 weist im Bereich des Endes 34 der Hülse 23 Einkerbungen 38 auf, die in Verbindung mit dem Ende 34 eine Anzeige 39 für die jeweilige Stellung der Welle 24 bilden. Die Welle 24 ist über den Anschlag 37 fest mit dem Gehäuse 4 verbunden.

Zur Durchführung der Untersuchung wird der Haltearm 20 in seinen Gelenken 22 derart verschwenkt und ausgerichtet, daß das Gehäuse 4 bzw. der Reizgeber 1 in Richtung derjenigen Körperstelle des Patienten ausgerichtet ist, auf die der Reizgeber 1 aufgelegt werden soll. In diesem Zustand, also wenn das Gehäuse 4 bzw. der Reizgeber 1 noch nicht auf der Haut des Patienten aufliegt, wird an der Anzeige 39 die Stellung der Welle 24 bzw. des Verlängerungsteils 31 zu der Hülse 23 abgelesen. Dies ist abhängig von der jeweiligen Lage des Gehäuses 4, also ob das Gehäuse 4 in Richtung des Pfeils 36 oder entgegen der Richtung des Pfeils 36, senkrecht oder schräg zu dem Pfeil 36 ausgerichtet ist. Dies begründet sich durch die in Richtung des Pfeils 36 wirkende Gewichtskraft. Entsprechend wird eine der Druckfedern 25 bzw. 26 belastet und die entsprechend andere Druckfeder 25 bzw. 26 entlastet. Das Gehäuse 4 und damit der Reizgeber 1 wird nun über den Haltearm 20 derart verstellt, daß das Gehäuse 4 bzw. die Auflagefläche 15 des Gehäuses 4 mit einem vorgegebenen Auflagedruck auf der Haut des Patienten zum Anliegen kommt. Der jeweils vorhandene Auflagedruck ist an der Anzeige 39 in Verbindung mit dem vorher abgelesenen Wert angezeigt. Damit ist sichergestellt, daß das Gehäuse 4 immer mit dem gleichen, vorher bestimmbaren Auflagedruck auf der entsprechenden Körperstelle des Patienten aufliegt. Weiterhin vorteilhaft ist, daß der Patient in Notfällen, beispielsweise wenn der Reizgeber 1 eine zu hohe Temperatur erreicht, sich von dem Reizgeber 1 befreien kann, indem er den Reizgeber 1 und das Gehäuse 4 entgegen des Pfeils 36 verschiebt. Durch die geschickte Anordnung des doppelt wirkenden Federsystems, also durch die Anordnung der Druckfedern 25 und 26, die jeweils in der einen Richtung wirken, wird die Welle 24 einerseits in einer mittleren Position gehalten und somit eine Bewegungsfreiheit in beiden Richtungen gewährleistet und andererseits der Vorteil erreicht, daß das Gehäuse 4 und der Reizgeber 1 in jeder beliebigen Position verwendbar ist. Ein und derselbe Auflagedruck wird also sowohl an der Hand eines Patienten, d. h. wenn das Gehäuse über Kopf angeordnet ist, als auch an einem Fuß des Patienten erreicht. Natürlich gilt das ebenso für jede x-beliebige andere, zwischen diesen Positionen liegende Stellung.

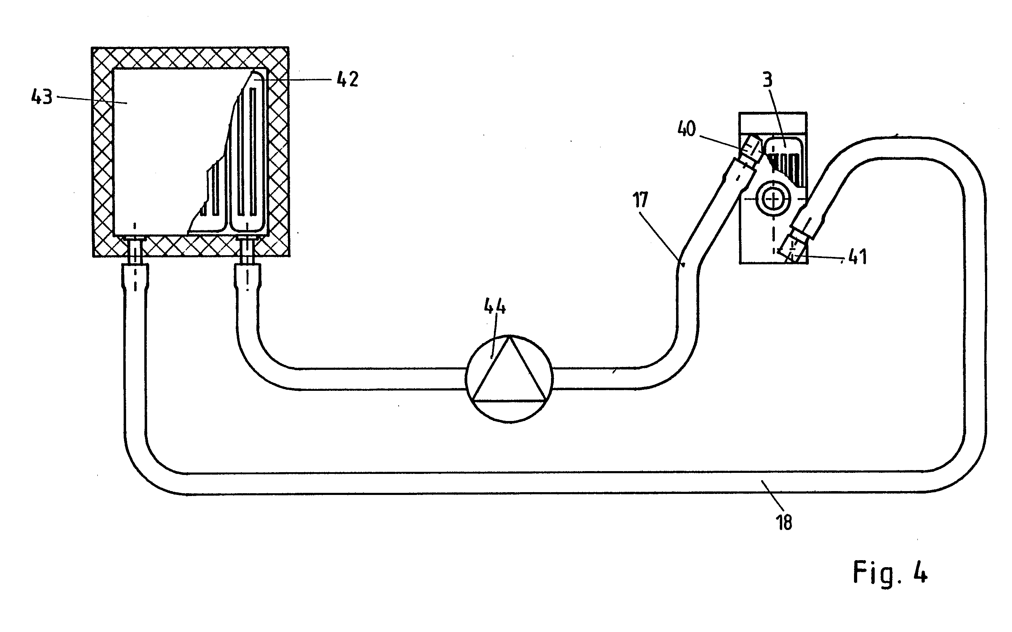

Figur 4 zeigt eine schematische Darstellung des Kühlkreislaufs der Stimulationsvorrichtung. Der Wärmetauscher 3 weist für die Abfuhrleitung 18 und die Zufuhrleitung 17, die aus flexiblem Material hergestellt sind, je einen Anschlußstutzen 40 und 41 auf. Am anderen Ende der Zufuhrleitung 17 bzw. Abfuhrleitung 18 ist ein zweiter Wärmetauscher 42 vorgesehen, der mit einem zweiten Peltier-Element 43 wärmeleitend in Verbindung steht. Das zweite Peltier-Element 43 ist dabei derart angeordnet bzw. wird derart gespeist, daß seine kalte Seite an dem Wärmetauscher 42 anliegt, so daß eine zusätzliche Kühlung erzielt wird. Zur Förderung der Kühlflüssigkeit ist zumindest in einer der Zufuhrleitung 17 oder der Abfuhrleitung 18 eine Pumpe 44 vorgesehen. Die Anordnung des zweiten Peltier-Elements 43 an dem zweiten Wärmetauscher 42 hat den Vorteil, daß in kurzer Zeit eine große Wärmemenge abgeführt werden kann und somit die Kühlleistung der Stimulationsvorrichtung erheblich erhöht wird. Dies ist insoweit wichtig, da die Abkühlzeit einen Einfluß auf die Untersuchungsergebnisse gezeigt hat.

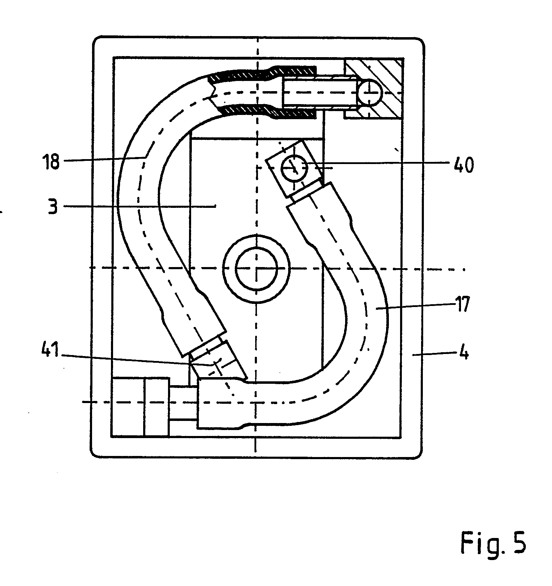

In der Figur 5 sind noch einmal die Anschlußstutzen 40 und 41 des Wärmetauschers 3 dargestellt. Die Anschlußstutzen 40 und 41 sind derart ausgebildet, daß die durch den Staudruck der Flüssigkeit hervorgerufenen Kräfte in einer Ebene senkrecht zu dem Pfeil 16, siehe Figur 2, wirken, also an der Zeichenebene entsprechend der Figur 5. Dies wird dadurch erreicht, daß die Anschlußstutzen 40 und 41 eine L-förmige Gestalt aufweisen, wobei der eine Schenkel der Anschlußstutzen 40 bzw. 41 in der Zeichenebene der Figur 5 liegt und als Einlaß benutzt wird, während der andere Schenkel senkrecht zu der Zeichenebene angeordnet ist und mit dem Wärmetauscher 3 in Verbindung steht. Dies hat den Vorteil, daß beispielsweise Schwankungen der Geschwindigkeit des Kühlmittels, die durch unterschiedliche Positionierung der Stimulationsvorrichtung auftreten können, nahezu keine Auswirkungen in Richtung der Achse 5 besitzen, also den Anpreßdruck des Reizgebers 1 nicht beeinflussen. Auch diese Maßnahme dient somit zur Konstanthaltung bzw. Reproduzierbarkeit des Anpreßdrucks des Reizgebers 1 und damit auch zur Sicherstellung der Reproduzierbarkeit der Untersuchungsergebnisse.

**Bezugszeichenliste:**

1 = Reizgeber
2 = Peltier-Element
3 = Wärmetauscher
4 = Gehäuse
5 = Achse
6 = Andruckeinrichtung
7 = Feder
8 = Anschlag
9 = Rändelschraube
10 = Pfeil
11 = Anzeigeeinrichtung
12 = Fenster
13 = Zeiger
14 = Überstand
15 = Auflagefläche
16 = Pfeil
17 = Zufuhrleitung
18 = Abfuhrleitung
19 = Ebene
20 = Haltearm
21 = zweite Andruckeinrichtung
22 = Gelenk

23 = Hülse
24 = Welle
25 = Druckfeder
26 = Druckfeder
27 = Auflage
28 = Auflage
29 = Auflage
30 = Auflage
31 = Verlängerungsteil
32 = Gewinde
33 = Gegengewinde
34 = Ende
35 = Anschlag
36 = Pfeil
37 = Anschlag
38 = Einkerbung
39 = Anzeige
40 = Anschlußstutzen
41 = Anschlußstutzen
42 = Wärmetauscher
43 = zweites Peltier-Element
44 = Pumpe

## Ansprüche

1. Stimulationsvorrichtung zur Bestimmung des Temperatur- und Schmerzempfindens eines Patienten mittels kutaner Kontaktstimulation, mit einem in einem Gehäuse angeordneten Reizgeber, der über ein Peltier-Element aufheizbar bzw. abkühlbar ist, dadurch gekennzeichnet, daß der Reizgeber (1) in dem Gehäuse (4) entlang einer Achse (5) beweglich angeordnet ist und in Ruhestellung gegenüber einer Auflagefläche (15) des Gehäuses (4) einen Überstand (14) aufweist, daß eine erste Andruckeinrichtung (6) für den Reizgeber (1) und eine zweite Andruckeinrichtung (21) für die Auflagefläche (15) des Gehäuses (4) vorgesehen ist, daß die erste Andruckeinrichtung (6) mit dem Reizgeber (1) derart zusammenwirkt, daß der Reizgeber (1) unter Aufbringung einer Kraft in Richtung der Achse (5) solange verschiebbar ist, bis der Überstand (14) zu Null wird, wobei die Kraft über die erste Andruckeinrichtung (6) einstellbar ist, und daß die zweite Andruckeinrichtung (21) mit dem Gehäuse (4) derart zusammenwirkt, daß die Auflagefläche (15) des Gehäuses (4) unter Aufbringung einer zweiten einstellbaren Kraft in Richtung der Achse (5) feststellbar ist.

2. Stimulationsvorrichtung nach Anspruch 1, dadurch gekennzeichnet, daß die erste Andruckeinrichtung (6) eine Feder (7) aufweist, die sich einerseits an dem Reizgeber (1) und andererseits an einem in dem Gehäuse (4) angeordneten Anschlag (8) abstützt.

3. Stimulationsvorrichtung nach Anspruch 2, dadurch gekennzeichnet, daß der im Gehäuse (4) angeordnete Anschlag (8) zur Verstellung der Größe der auf den Reizgeber (1) wirkenden Kraft vorzugsweise koaxial zur Bewegungsrichtung des Reizgebers (1) verstellbar ist.

4. Stimulationsvorrichtung nach einem der Ansprüche 1 bis 3, dadurch gekennzeichnet, daß eine die auf den Reizgeber (1) wirkende Kraft bzw. die Stellung des Anschlags (8) wiedergebende Anzeigeeinrichtung (11) vorgesehen ist.

5. Stimulationsvorrichtung nach Anspruch 1, dadurch gekennzeichnet, daß die zweite Andruckeinrichtung (21) einen in allen Richtungen schwenk- und verstellbaren Haltearm (20) aufweist, und daß das Gehäuse an der zweiten Andruckeinrichtung (21) gelagert ist.

6. Stimulationsvorrichtung nach Anspruch 5, dadurch gekennzeichnet, daß die zweite Andruckeinrichtung (21) eine mit dem Gehäuse (4) starr verbundene Welle (24) aufweist, daß die Welle (24) in einer an dem Haltearm (20) befestigbaren Hülse (23) beweglich geführt ist, und daß eine die Welle (24) in einer vorzugsweise mittleren Position fixierende Haltevorrichtung angeordnet ist.

7. Stimulationsvorrichtung nach Anspruch 6, dadurch gekennzeichnet, daß die Haltevorrichtung derart ausgebildet und angeordnet ist, daß die Welle (24) aus der fixierten Position unter Aufbringung einer Kraft verschiebbar ist.

8. Stimulationsvorrichtung nach Anspruch 6 oder 7, dadurch gekennzeichnet, daß die Haltevorrichtung vorzugsweise zwei Druckfedern (25, 26) aufweist, daß jeder Druckfeder (25, 26) eine Auflage (27, 28) an der Hülse (23) und eine zweite Auflage (29, 30) an der Welle (24) zugeordnet sind, und daß bei Belastung der einen Druckfeder (25, 26) die andere entlastet wird und umgekehrt.

9. Stimulationsvorrichtung nach Anspruch 8, dadurch gekennzeichnet, daß die Haltevorrichtung eine Anzeige (39) aufweist, von der die Größe und die Richtung der auf den Druckfedern (25, 26) wirkenden Belastung ablesbar ist.

10. Stimulationsvorrichtung nach einem der Ansprüche 1 bis 9, dadurch gekennzeichnet, daß dem Peltier-Element (2) ein flüssigkeitsgekühlter Wärmetauscher (3) zugeordnet ist, und daß ein zweites, die Flüssigkeit des Wärmetauschers (3, 42) kühlendes Peltier-Element (43) vorgesehen ist.

Fig. 1

Fig. 2

Fig. 3

Fig. 4

EP 0 347 640 A1

Fig. 5

**Europäisches Patentamt**

# EUROPÄISCHER RECHERCHENBERICHT

Nummer der Anmeldung

EP 89 11 0180

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE-A-3 309 093 (K. MOSLER) * Zusammenfassung; Seiten 1,2; Ansprüche 1-3,7; Seite 4, Zeilen 21-29; Seite 7, Zeile 21 - Seite 8, Zeile 14; Seite 9, Zeilen 24-31; Figuren 1-3 * --- | 1,3 | A 61 B 5/00 A 61 B 5/10 |
| A | US-A-3 570 312 (F. KREITH) * Spalte 1, Zeile 69 - Spalte 2, Zeile 31; Spalte 4, Zeilen 18-49; Figuren 1,2 * --- | 1,2-4 | |
| A | FR-A-2 583 974 (E. LAVIT) * Zusammenfassung; Seite 2, Zeilen 8-24; Seite 3, Zeilen 12-23; Seite 4, Zeilen 10-13,25-30; Seite 5, Zeile 26 - Seite 6, Zeile 32; Seite 7, Zeilen 20-26; Figur 2 * ----- | 1-3 | |

**RECHERCHIERTE SACHGEBIETE (Int. Cl.4)**

A 61 B
A 61 G
A 61 H

Der vorliegende Recherchenbericht wurde für alle Patentansprüche erstellt

| Recherchenort | Abschlußdatum der Recherche | Prüfer |
|---|---|---|
| DEN HAAG | 08-09-1989 | RIEB K.D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)